# EUROPÄISCHE PATENTSCHRIFT

(11) **EP 1 325 704 B1**
(45) Veröffentlichungstag und Bekanntmachung des Hinweises auf die Patenterteilung: **01.03.2006**
(21) Anmeldenummer: 02027176.3
(22) Anmeldetag: 05.12.2002
(51) Int. Cl.: A61B 1/00, A61B 17/32

(54) **Hysteroskop mit Wechselschaftsystem**
Hysteroscope with interchangable shafts
Hystéroscope à tiges interchangeables

(30) Priorität: 28.12.2001 DE 10164384
(43) Veröffentlichungstag der Anmeldung: 09.07.2003
(73) Patentinhaber: Richard Wolf GmbH, 75438 Knittlingen (DE)
(72) Erfinder: Boebel, Manfred, 75245 Bauschlott (DE); Brüstle, Sybille, 75447 Sternenfels (DE)
(74) Vertreter: Hemmer, Arnd

(56) Entgegenhaltungen:
- DE-A- 3 319 049
- DE-A- 3 341 385
- DE-A- 4 445 105
- DE-A- 10 009 020
- DE-U- 29 720 643
- US-A- 5 582 575

## Beschreibung

Die Erfindung betrifft ein Endoskop, ein Endoskopset sowie einen Satz von Schäften für ein Endoskop.

Bei Endoskopen, beispielsweise Hysteroskopen, ist es wünschenswert, den Endoskopschaft mit einem möglichst kleinen Außendurchmesser auszubilden. Der minimale Außendurchmesser wird jedoch insbesondere durch die Größe der einzuführenden Instrumente vorgegeben. Bei bekannten Endoskopen, wie sie z. B. aus DE 297 20 643 U1, DE 100 09 020 A1, DE 44 05 720 C1 oder DE 3319049 A1 bekannt sind, beginnt der Operateur üblicherweise mit einem Instrument und Endoskop, welche den geringstmöglichen Durchmesser aufweisen. Bei Bedarf wird dieses Instrument durch ein Instrument, welches einen größeren Durchmesser besitzt, ausgetauscht. Dies kann beispielsweise dann der Fall sein, wenn größere Gewebemengen aus der Körperhöhle entfernt werden müssen. In diesem Fall ist dann auch ein Endoskop mit einem größeren Instrumentenkanaldurchmesser erforderlich. Da bei den bekannten Endoskopen die in deren Inneren angeordneten Kanäle in ihrem Durchmesser derart aufeinander abgestimmt sind, dass der freie Raum im Endoskopschaft optimal ausgenutzt wird, müssen mehrere Endoskope mit unterschiedlichen Abmessungen bereitgehalten werden. Die Bereitstellung mehrerer Endoskope verschiedener Größe ist mit hohen Kosten verbunden.

Es ist Aufgabe der Erfindung, ein Endoskop bzw. ein Endoskopset zu schaffen, welches die Bereitstellung mehrerer Endoskope mit unterschiedlichen Durchmessern für eine Operation vermeidet.

Diese Aufgabe wird durch ein Endoskop mit den im Anspruch 1 angegebenen Merkmalen gelöst. Bevorzugte Ausführungsfomen ergeben sich aus den jeweiligen Unteransprüchen.

Das erfindungsgemäße Endoskop besteht u.a. aus einer Arbeitsoptik, die an ihrem proximalseitigen Ende ein Optikgehäuse mit einem vorzugsweise seitlich im Winkel zur Instrumentenlängsachse angeordneten Einblick aufweist. Das proximalseitige Ende des Endoskops bzw. der Arbeitsoptik ist dabei dasjenige Ende, welches dem Operateur zugewandt ist, während das distale Ende des Endoskops in die Körperöffnung eingeführt wird. Mit dem Optikgehäuse ist ein Außenschaft lösbar verbunden. Der Außenschaft ist derjenige Endoskopschaft, welcher den maximalen Außendurchmesser des in eine Körperöffnung einzuführenden Instrumententeils definiert. Ferner ist ein Innenschaft vorgesehen, welcher im Inneren des Außenschaftes parallel zu diesem verläuft und mit dem Optikgehäuse ebenfalls lösbar verbunden ist. Bei dem erfindungsgemäß aufgebauten Endoskop handelt es sich beispielsweise um ein Hysteroskop. Der Außenschaft und der Innenschaft können von dem Optikgehäuse abgenommen werden. Der Innenschaft erstreckt sich vorzugsweise in Längsrichtung des Instruments durch das Optikgehäuse. Zum Trennen von dem Optikgehäuse wird der Innenschaft aus diesem vorzugsweise nach hinten herausgezogen. Gemäß der Erfindung ist es möglich, unterschiedliche Außen- und Innenschäfte mit ein und demselben Optikgehäuse bzw. ein und derselben Arbeitsoptik zu verbinden. Somit ist es für eine Operation, bei welcher Schäfte mit unterschiedlich großen Durchmessern benötigt werden, nicht mehr erforderlich, mehrere vollständige Endoskope bereitzustellen. Es müssen lediglich die Endoskopschäfte gewechselt werden. Auf diese Weise können die Anschaffungskosten erheblich reduziert werden. Ferner begünstigt die Zerlegbarkeit des Endoskops die Reinigung und Desinfektion. Der Innenschaft des Endoskops bildet einen Instrumentenkanal, durch welchen verschiedenartige Instrumente zur Durchführung einer Operation und/oder Diagnose in den Körper eingeführt werden können. Abhängig von der Größe der einzusetzenden Instrumente können Innenschäfte mit unterschiedlichen Durchmessern mit dem Optikgehäuse verbunden werden. Abhängig vom Außendurchmesser des verwendeten Innenschaftes muss ein Außenschaft mit passendem Durchmesser ausgewählt werden und austauschbar mit dem Optikgehäuse verbunden werden.

Der Innenschaft ist aus dem Außenschaft entnehmbar ausgebildet. Diese Anordnung ermöglicht, dass Außen- und Innenschäfte unabhängig voneinander gewechselt werden können. Außen- und Innenschaft bilden keine Einheit, sondern können voneinander getrennt werden. So ist es auch möglich, ein und denselben Außenschaft mit unterschiedlichen Innenschäften oder ein und denselben Innenschaft mit unterschiedlichen Außenschäften zu verwenden. Das derartig aufgebaute Endoskop bietet somit eine Vielzahl von Kombinationsmöglichkeiten unterschiedlicher Schäfte, was eine vielseitige Anpassbarkeit des Endoskops an unterschiedliche Einsatzzwecke ermöglicht. Um die Zuordnung zwischen einem Außenschaft und einem passenden bzw. zugehörigen Innenschaft zu erleichtern, können die vorzugsweise paarweise aufeinander abgestimmten Schäfte beispielsweise farblich oder in anderer geeigneter Weise gekennzeichnet werden. Obwohl die Verwendung und der Austausch der mit der Arbeitsoptik verwendeten Schäfte im Allgemeinen paarweise erfolgt, besteht grundsätzlich die Möglichkeit, einen Außenschaft mit Innenschäften, die einen kleineren Durchmesser aufweisen, zu verwenden. Ein gleichzeitiger Austausch des Außenschaftes unterbleibt dann, wenn zur Ausführung eines nachfolgenden Operationsschrittes wieder auf einen Innenschaft mit größerem Durchmesser zurückgegriffen werden muss.

Zweckmäßigerweise ist im Inneren des Außenschaftes ein Optikschaft angeordnet, welcher sich in Längsrichtung des Außenschaftes erstreckt. Der Optikschaft beinhaltet das optische System des Endoskops und ist vorzugsweise fest mit dem Optikgehäuse verbunden. Somit bleibt beim Austausch der wechselbaren Außen- und Innenschäfte der Optikschaft immer derselbe, so dass dieser nicht in Abhängigkeit der verwendeten Außen- und Innenschäfte getauscht werden muss. Das erfindungsgemäße Endoskop ermöglicht es somit, eine einzige Arbeitsoptik mit einer Vielzahl von unterschiedlich dimensionierten Außen- und Innenschäften zu verwenden. Dies reduziert die Anschaffungskosten erheblich, da es nicht mehr erforderlich ist, eine Mehrzahl vollständiger Endoskope mit jeweils einer eigenen Arbeitsoptik vorzuhalten. Bei der Abstimmung der Durchmesser von Außen- und Innenschaftzueinander ist davon auszugehen, dass die Abmessungen der Endoskopoptik, d. h. vor allem Querschnittsgeometrie und Länge der Optik, festliegen

Vorzugsweise ist in dem Optikschaft ein Lichtleiter zur Beleuchtung angeordnet. Dieser wird in bekannter Weise beispielsweise durch Lichtleitfasern bzw. Glasfasern gebildet.

Ferner kann in dem Optikschaft eine Bildübertragungsoptik angeordnet sein. Diese kann ebenfalls aus einem Lichtleiter Lichtleitfasern bestehen, welche(r) ein Bild durch den Optikschaft zu der Optik des Optikgehäuses überträgt. Alternativ kann anstelle einer optischen Bildaufnahme und Bildübertragung eine elektronische Bildaufnahme und -übertragung unter Verwendung eines CCD-Elementes oder dergleichen erfolgen.

Auch der Optikschaft kann in einer alternativen Ausführungsform mit dem Optikgehäuse lösbar verbunden sein. Dies begünstigt die Reinigung und Desinfektion des Endoskops. Ferner ist es auch möglich, unterschiedliche Optiken mit ein und demselben Optikgehäuse zu verwenden.

Zwischen dem Innenschaft und einer Innenwandung des Außenschaftes ist vorzugsweise ein Freiraum ausgebildet. Ein solcher Freiraum kann beispielsweise als Spülkanal verwendet werden. Er ist dazu bevorzugt im Optikgehäuse mit entsprechenden Schlauchanschlüssen verbunden. Ein solcher Freiraum kann beispielsweise dadurch entstehen, dass in einem Außenschaft mit im Wesentlichen kreisförmigem Querschnitt ein Innenschaft mit ebenfalls im Wesentlichen kreisförmigem Querschnitt, welcher einen geringeren Durchmesser aufweist, verwendet wird. Der mit dem Optikgehäuse verbindbare Innenschaft dient vorzugsweise neben der Aufnahme von Hilfsinstrumenten ebenfalls als Spülkanal zum Zuführen einer Spülflüssigkeit. Das proximalseitige Optikgehäuse weist zu diesem Zweck einen vorzugsweise absperrbaren Schlauchanschlussstutzen bzw. Instrumentenhahn auf. Auch nach Einführen eines Hilfsinstrumentes ist der im Innenschaft verbleibende Freiraum ausreichend, um die erforderliche Menge der in die Körperhöhle einzuleitenden Spülflüssigkeit zuführen zu können.

Auch zwischen dem sich distalwärts erstreckenden Optikschaft und der Innenwandung des Außenschaftes kann ein Freiraum verbleiben. Auch wenn in dem Außenschaft ein Optikschaft angeordnet ist, kann der gesamte Freiraum, welcher zwischen den Außenwandungen von Innenschaft und Optikschaft sowie der Innenwandung des Außenschaftes verbleibt, zu Spülzwecken, insbesondere zum Abführen der Spülflüssigkeit verwendet werden. Auf diese Weise sind, ohne zusätzliche Spülkanäle vorsehen zu müssen, die Querschnitte der Schäfte optimal ausgenutzt, so dass ein Endoskopschaft mit minimalem Durchmesser bereitgestellt werden kann.

Bevorzugt werden der Außenschaft und der Innenschaft über Kupplungskegelverbindungen mit dem Optikgehäuse lösbar verbunden. Solche Kupplungskegelverbindungen sind im Stand der Technik bekannt und ermöglichen eine leichte und feste Fixierung der Schäfte an dem Optikgehäuse. Ferner ermöglichen sie eine gute Abdichtung der durch die Schäfte gebildeten Kanäle.

Weiterhin betrifft die Erfindung ein Endoskopset, welches ein Optikgehäuse und zumindest einen Außenschaft und zumindest einen Innenschaft umfasst. Der Außenschaft ist lösbar mit dem Optikgehäuse verbindbar, so dass er ausgetauscht werden kann. Der Innenschaft ist im Inneren des Außenschaftes, welcher im Wesentlichen rohrförmig ausgebildet ist, anordbar und ebenfalls mit dem Optikgehäuse lösbar verbindbar. Der Innenschaft ist ebenfalls im Wesentlichen rohrförmig ausgebildet und bildet einen Instrumentenkanal, durch welchen Instrumente in das Körperinnere zur Durchführung einer Operation oder Diagnose eingeführt werden können. Die lösbare Anordnung von Außenschaft und Innenschaft ermöglicht es, diese Schäfte leicht auszutauschen. Es wird somit ein Endoskop geschaffen, welches es ermöglicht, unterschiedlich ausgestaltete, d.h. insbesondere unterschiedlich dimensionierte Außen- und Innenschäfte mit ein und demselben Optikgehäuse zu verwenden. Beispielsweise können Außen- und Innenschäfte von unterschiedlichem Durchmesser und unterschiedlicher Geometrie verwendet werden, um das Endoskop an unterschiedliche Einsatzzwecke anzupassen. Ferner begünstigt die Lösbarkeit der Außen- und Innenschäfte vom Optikgehäuse die Reinigung und Desinfektion des Endoskops. Bevorzugt handelt es sich bei dem Endoskopset um ein Hysteroskopset.

Der Innenschaft ist vorzugsweise im Inneren des Außenschaftes derart anordbar, dass er exzentrisch zu dem Außenschaft verläuft. Auf diese Weise wird ein möglichst großes freies Lumen im Inneren des Außenschaftes geschaffen.

Bevorzugt weist das Endoskopset mehrere austauschbare Innenschäfte auf. Die Innenschäfte können unterschiedlich dimensioniert und an verschiedene Einsatzzwecke angepasst sein. Zur Verwendung des Endoskops wird der gewünschte Innenschaft mit dem Optikgehäuse verbunden.

Die mehreren Innenschäfte weisen vorzugsweise unterschiedliche Querschnittsgrößen auf. Auf diese Weise können Instrumentenkanäle mit unterschiedlich großem Querschnitt zur Verfügung gestellt werden. Es ist beispielsweise möglich, eine Untersuchung bzw. Operation mit einem Instrumentenkanal mit geringem Querschnitt zu beginnen. Wird dann während der Behandlung ein größerer Instrumentenkanal benötigt, kann an das Optikgehöuse mit einem Innenschaft mit größerem Querschnitt bzw. Durchmesser ausgestattet werden.

Vorzugsweise sind ebenfalls mehrere austauschbare Außenschäfte in dem Endoskopset vorgesehen. Die Außenschöfte sind an unterschiedliche Einsatzzwecke angepasst und können je noch beabsichtigter Verwendung leicht an dem Optikgehäuse befestigt und wieder gelöst werden.

Bevorzugt weisen die Außenschäfte unterschiedliche Querschnittsgrößen auf. So kann sehr leicht das Endoskop an unterschiedliche Innenkanäle bzw. Innenschäfte angepasst werden, ohne dass vollständige Endoskope mit unterschiedlichen Endoskopschaftdurchmessern bereitgehalten werden müssen. Die Außenschöfte und Innenschäfte sind vorzugsweise paarweise aufeinander abgestimmt, so dass jedem Innenschaft ein entsprechend dimensionierter Außenschaft zugeordnet ist. Die einfache Koppelbarkeit und Trennbarkeit mit bzw, von der Arbeitsoptik bzw. dem Optikgehäuse ermöglicht, dass während einer Behandlung die unterschiedlich großen Schäfte leicht gegeneinander ausgetauscht werden können. Der Aufbau des Endoskopsets ermöglicht somit eine sehr vielseitige Verwendung und Anpassbarkeit an unterschiedliche Einsatzzwecke, ohne dass mehrere unterschiedliche Endoskope bereitgehalten werden müssen.

In dem Endoskopset ist zweckmäßigerweise ferner ein Optikschaft vorgesehen, welcher im Inneren des Außenschaftes anordbar ist. Der Optikschaft ist Teil der Arbeitsoptik und erstreckt sich distalwärts von dem Optikgehäuse und ist vorzugsweise exzentrisch im Inneren des Außenschaftes anordbar. DieArbeitsoptik kann in bekannter Weise eine Bildübertragungseinrichtung sowie eine Beleuchtungseinrichtung beinhalten. Sowohl die Beleuchtungs- als auch die Bildübertragungseinrichtung kann aus Lichtleitern, beispielsweise Glasfasern, bestehen, welche Licht zur Beleuchtung von dem Optikgehäuse und insbesondere von einem an dem Optikgehäuse vorhandenen Lichtleitkabelanschlussstutzen zum distalen Ende des Endoskopschaftes leiten und ein aufgenommenes Bild vom distalen Ende zum Optikgehäuse übertragen. Dort ist vorzugsweise seitlich im Winkel zur Längsachse ein Okular angeordnet, mit welchem das Bild betrachtet werden kann. Alternativ kann anstelle einer optischen Bildübertragung eine elektronische Bildübertragung unter Verwendung eines im distalen Schaftende angeordneten CCD-Elementes oder dergleichen vorgesehen sein. Anstelle der Lichtleiter kann auch ein Beleuchtungselement direkt am distalen Ende im Optikschaft angeordnet werden. Bevorzugt ist in dem Optikschaft die Bildübertragungsoptik bzw. das Bildbündel zentral angeordnet und wird von den Lichtleitern zur Beleuchtung umgeben.

Der Optikschaft ist vorzugsweise fest mit dem Optikgehäuse verbunden. Da es bei wechselnder Verwendung von Innen- und Außenschäften mit unterschiedlichen Querschnittsgrößen nicht erforderlich ist, ebenfalls eine andere Arbeitsoptik zu verwenden, kann auf einen Austausch der Optik in den meisten Fällen verzichtet werden. Beim Austausch des Außenschaftes wird dieser über den Optikschaft geschoben und dann mit dem Optikgehäuse verbunden. Die Größe des verwendeten Optikschafts stellt dabei eine Begrenzung für den minimalen Durchmesser des Außenschaftes dar.

Alternativ kann jedoch auch der Optikschaft lösbar mit dem Optikgehäuse verbindbar sein. So kann auch die Arbeitsoptik an unterschiedliche Einsatzzwecke angepasst werden. Ferner wird die Reinigung und Desinfizierbarkeit des Endoskops verbessert.

Der vorgesehene Optikschaft weist zweckmäßigerweise eine Querschnittsgröße auf, welche geringer ist als ein Innenquerschnitt jedes Außenschaftes. Dies ermöglicht, dass sämtliche Außenschäfte des Endoskopsets mit ein und demselben Optikschaft bzw. derselben Arbeitsoptik verwendet werden können.

Ferner ist gemäß der Erfindung ein Satz von Schäften für ein Endoskop vorgesehen. Dieser Satz von Schäften für ein Endoskop besteht aus einer Mehrzahl von Schäften mit unterschiedlicher Querschnittsgröße, wobei sämtliche Schäfte an einem Ende eine identisch ausgebildete Aufnahme zum Verbinden mit der Arbeitsoptik bzw. dem Optikgehäuse aufweisen. Abgesehen von der identisch ausgebildeten Aufnahme können die Schäfte an unterschiedliche Einsatzzwecke angepasst sein, insbesondere können sie unterschiedliche Innen- und/oder Außenquerschnitte aufweisen. Es ist somit möglich, einen Satz von Endoskopschäften mit unterschiedlichen Durchmessern bzw. Querschnittsgrößen zur Verfügung zu stellen, welche je nach Einsatzzweck mit einer Arbeitsoptik bzw. einem Optikgehäuse verbunden werden können. Dabei kann jeder der Schäfte des Satzes aufgrund der identischen bzw. standardisierten Aufnahme- bzw. Anschlussgeometrie mit derselben Arbeitsoptik bzw. demselben Optikgehäuse verwendet werden.

Bevorzugt ist eine Mehrzahl von Außen- und Innenschäften vorgesehen, wobei die Innenschäfte so ausgebildet sind, dass jeder Innenschaft im Inneren von zumindest einem der Außenschäfte anordbar ist und die Innenschäfte und Außenschäfte jeweils identisch ausgebildete Aufnahmen aufweisen. Aufgrund der identisch ausgebildeten Aufnahmen können jeder Innenschaft und jeder Außenschaft lösbar mit ein und derselben Arbeitsoptik bzw. demselben Optikgehäuse verbunden werden. Bevorzugt ist zu jedem Innenschaft zumindest ein passender Außenschaft vorgesehen. Es ist somit möglich, an ein und derselben Arbeitsoptik bzw. demselben Optikgehäuse unterschiedlich dimensionierte Instrumentenkanäle anzuschließen. Beispielsweise kann zu Beginn einer Untersuchung bzw. Operation zunächst ein Innenschaft mit geringem Querschnitt gewählt werden, welcher einen Instrumentenkanal mit minimalem Durchmesser zur Verfügung stellt. Dabei kann ein zugehöriger Außenschaft mit minimalem Querschnitt eingesetzt werden. Stellt sich dann während der Behandlung heraus, dass ein größerer Instrumentenkanal zum Einführen eines größeren Instruments erforderlich ist, kann der Innenschaft gegen einen Innenschaft mit größerem Querschnitt ausgetauscht werden. Da ein Innenschaft mit größerem Innenquerschnitt meist auch einen größeren Außenquerschnitt aufweist, ist es dann möglich, einen entsprechend größeren Außenschaft zu verwenden und mit der Arbeitsoptik bzw. dem Optikgehäuse zu verbinden. Ein solcher Satz von Innen- und Außenschäften, welche vorzugsweise paarweise aufeinander abgestimmt sind, ermöglicht somit eine leichte Anpassbarkeit des Endoskops an unterschiedliche Einsatzzwecke. Es ist somit nicht erforderlich, für jeden erforderlichen Schaftquerschnitt ein separates Endoskop mit einer eigenen Arbeitsoptik zur Verfügung zu stellen.

Vorzugsweise ist in dem Satz von Schäften zumindest ein Paar von zusammengehörenden Innen- und Außenschöften vorgesehen. In dem Paar ist der Außenschaft in seiner Dimensionierung auf den zugehörigen Innenschaft abgestimmt.

Zweckmäßigerweise sind der Innen- und der Außenschaft des Paares als zusammengehörend, beispielsweise durch eine Farbmarkierung gekennzeichnet. Dies ermöglicht auch in einer Vielzahl von Schaftpaaren leicht die zusammengehörenden Innen- und Außenschäfte zu finden.

Nachfolgend wird die Erfindung beispielhaft anhand der beigefügten Figuren beschrieben. In diesen zeigt:
- Fig. 1: eine Gesamtansicht des erfindungsgemäßen Endoskops,
- Fig. 2: einen Längsschnitt des in Fig. 1 gezeigten Endoskops,
- Fig. 3: einen Längsschnitt des Innenschaftes des Endoskops gemäß Fig. 1 und 2 und
- Fig. 4A - 4E: Querschnittsansichten des Endoskopschaftes bei Verwendung von Innen- und Außenschäften mit unterschiedlichen Querschnittsgrößen.

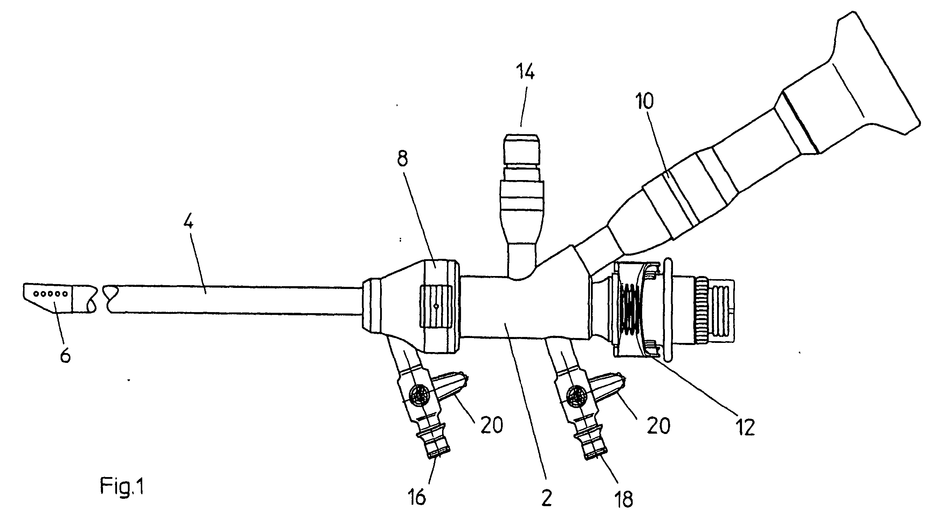

Bei dem in Fig. 1 gezeigten Beispiel des erfindungsgemäßen Endoskops handelt es sich um ein Hysteroskop. Das Endoskop bzw. Hysteroskop weist ein Optikgehäuse 2 und einen angesetzten Endoskopschaft bzw. Außenschaft 4 auf. Der Außenschaft 4 ist an seinem distalen Ende 6, d. h. an seinem dem Optikgehäuse 2 abgewandten Ende, offen ausgebildet. In dem rohrförmigen Außenschaft 4 werden die erforderlichen Endoskopschäfte bzw. -kanäle wie Instrumentenkanol, Spülkanal und Optikschaft angeordnet. Diese Schäfte bzw. Kanäle können an dem geöffneten distalen Ende 6 austreten bzw. sind ebenfalls zu desem hin geöffnet. Am proximalen Ende 8 des Außenschaftes 4 ist eine Kupplungseinrichtung ausgebildet, über die der Außenschaft 4 mit dem Optikgehäuse 2 bzw. der Arbeitsoptik lösbar verbunden ist. An dem Optikgehäuse 2 ist in bekannter Weise eine Arbeitsoptik 10 ausgebildet. Das Okular 10 der Arbeitsoptik ist zur Längsachse des Optikschaftes der Arbeitsoptik, d. h. zur Längsachse des Außenschaftes 4, abgewinkelt, so dass in Längsrichtung des Endoskops bzw. Außenschaftes 4 am proximalen Ende des Optikgehäuses 2 ein Innenschaft 12 eingesetzt werden kann. Der Innenschaft 12 ist vorzugsweise zum hinteren bzw. proximalen Ende des Optikgehäuses 2 hin geöffnet, so dass durch den Instrumentenkanal, welcher durch den Innenschaft 12 gebildet wird, Instrumente in das Endoskop eingeführt werden können. Ferner ist an dem Optikgehäuse ein Lichtleitkabelanschlussstutzen 14 vorgesehen. An diesen Lichtleitkabelanschlussstutzen 14 wird in bekannter Weise eine Beleuchtungseinrichtung angeschlossen. Ferner sind an dem proximalen Ende 8 des Außenschaftes 4 sowie an dem Optikgehäuse 2 zwei Schlauchanschlussstutzen 16 und 18 vorgesehen. Die Schlauchanschlussstutzen 16 und 18 weisen Absperrhähne 20 auf und sind mit Spülkanälen im Inneren des Endoskops verbunden. Über die Schlauchanschlussstutzen 16 und 18 werden die Spülkanäle beispielsweise mit einer Saug-/Spülpumpe zum Zu- und Abführen einer Spülflüssigkeit verbunden.

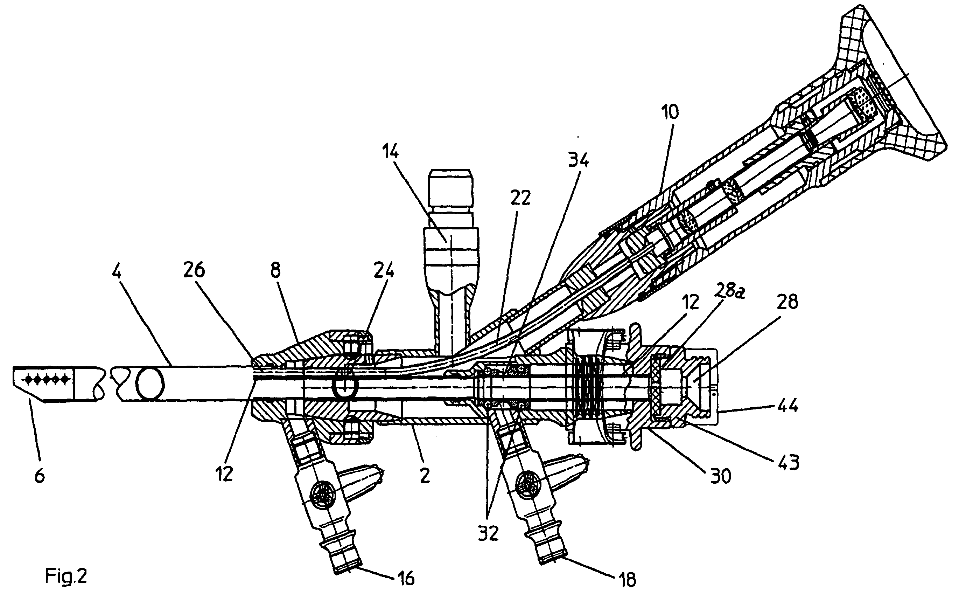

Fig. 2 zeigt eine Schnittsansicht des in Fig. 1 gezeigten Endoskops. Die abgewinkelte Endoskopoptik 10 sowie der Lichtleitkabelanschlussstutzen 14 sind in bekannter Weise ausgebildet, weshalb auf eine nähere Beschreibung verzichtet wird. Das Okular 10 ist mit einem Bildbündel oder einer einzelnen Lichtleit- bzw. Bildfaser 22 zur Bildübertragung verbunden. In dem Lichtleitkabelanschlussstutzen 14 sind die proximalseitigen Enden der Lichtleitfasern bzw. das proximalseitige Ende des Lichtleitelementes festgelegt. Das Bildbündel bzw. die einzelne Bildfaser 22 sowie die Lichtleitfasern bzw. Lichtleitelemente 24 zur Beleuchtung sind gemeinsam in dem sich distalwärts erstreckenen Optikschaft 26 festgelegt, welcher sich in Längsrichtung des Außenschaftes 4 parallel zu diesem erstrec kt. Der Optikschaft 26 weist einen im Wesentlichen halbkreisförmigen Querschnitt auf, wobei das Bildbündel 22 zur Bildübertragung im Wesentlichen einen kreisförmigen Querschnitt aufweist. Das Bildbündel 22 ist im Wesentlichen zentral in dem Optikschaft 26 angeordnet, während die Lichtleitfasern 24 zur Beleuchtung umfänglich bzw. seitlich zum Bildbündel 22 in dem Optikschaft 26 angeordnet sind. Der Radius des im Wesentlichen halbkreisförmigen Optikschafts 26 ist geringer als der Radius des Außenschaftes 4, welcher im Wesentlichen einen kreisförmigen Querschnitt aufweist. Der Optikschaft 26 ist exzentrisch in dem Außenschaft 4 in dessen oberen Bereich angeordnet und kann am Innenumfang des Außenschaftes 4 anliegen.

Der Außenschaft 4 weist an seinem proximalen Ende einen Schnellspannverschluss 8 auf. Der Schnellspannverschluss 8 ist in bekannter Weise als Kegelspannverschluss ausgebildet. Im Inneren des Schnellspannverschlusses 8 ist eine konische Innenumfangsfläche ausgebildet, welche auf eine korrespondierende konische Außenumfangsfläche an dem Optikgehäuse 2 dichtend aufgesetzt wird. Federbelastete Kugeln dienen zur Verrastung der beiden Kegelelemente miteinander. An dem Schnellspannverschluss 8 ist ferner der Schlauchanschlussstutzen 16 angebracht. Der Schlauchanschlussstutzen 16 ist mit dem Innenraum des Außenschaftes 4 verbunden. Der Innenraum des Außenschaftes 4, welcher den eingesetzten Innenschaft 12 sowie den Optikschaft 26 umgibt, wird als ein erster Spülkanal verwendet.

In das Innere des Optikgehäuses 2 sowie des Außenschaftes 4 ist ein Innenschaft 12 eingesetzt. Der Innenschaft 12 ist im Wesentlichen rohrförmig ausgebildet und weist einen im Wesentlichen halbkreisförmigen Querschnitt auf. Die zentrale Längsachse des Innenschaftes 12 ist versetzt zu der Längsachse des Außenschaftes 4 angeordnet, so dass der Innenschaft 12 sowie der Optikschaft 26 neben- bzw. übereinander im Inneren des Außenschaftes 4 angeordnet werden können. Dabei sind die planen Flächen des halbkreisförmigen Optikschaftes 26 und des halbkreisförmigen Innenschaftes 12 einander zugewandt.

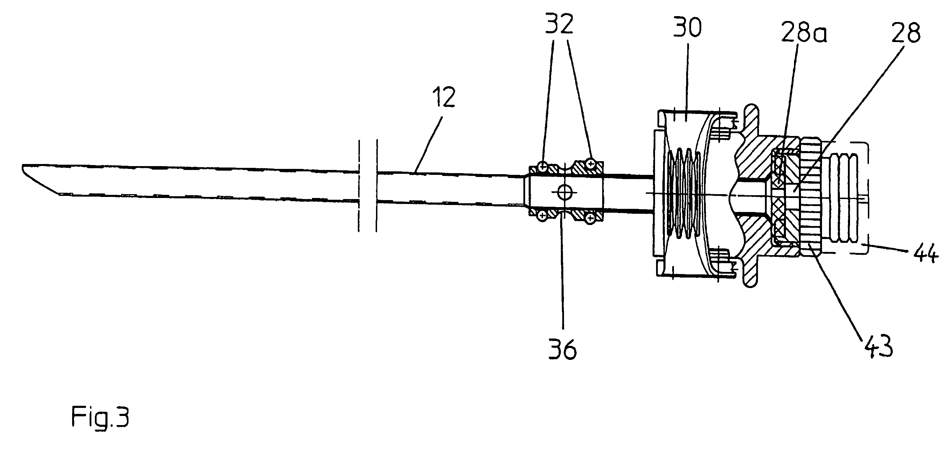

Der Innenschaft 12 wird vom proximalen, d. h. hinteren Ende des Optikgehäuses 2 in dieses und den Außenschaft 4 eingeführt. Der Innenschaft 12 erstreckt sich vorzugsweise über die gesamte Länge des Außenschaftes 4 und ist zu dessen offenen Ende 6 hin geöffnet. Der Innenschaft 12 definiert den Instrumentenkanal des Endoskops, durch den Instrumente eingeführt werden können. Der Innenschaft 12 weist an seinem proximalen bzw. hinteren Ende eine Öffnung 28 auf, durch die die Instrumente In das Endoskop bzw. den Instrumentenkanal eingeführt werden. Ebenfalls am hinteren bzw. proximalen Ende des Innenschaftes 12 ist ein Kupplungselement 30 zur dichtenden Verbindung des Innenschaftes 12 mit dem Optikgehäuse 2 ausgebildet. Das Kupplungselement 30 ist ebenfalls als Kupplungskegelverbindung mit einem Schnellspannverschluss ausgebildet. Im Inneren des Kupplungselementes 30 an dem Innenschaft 12 ist eine konische Ausnehmung ausgebildet, welche auf eine kegelförmige bzw. konische Fläche an dem proximalen Ende des Optikgehäuses 2 dichtend aufgesetzt wird. Ferner sind an dem Innenschaft 12 zwei Umfangsdichtungen 32 vorgesehen, welche einen Umfangsraum 34 gegenüber dem Inneren des Optikgehäuses 2 abdichten. Als Umfangsdichtungen 32 können beispielsweise O-Ringe vorgesehen sein. Der Umfangsraum 34 ist zum Inneren des Innenschaftes 12 hin geöffnet. Die Öffnung des Schlauchanschlussstutzens 18 ist derart platziert, dass sich der Schlauchanschlussstutzen 18 zu dem Umfangsraum 34 hin öffnet. Somit ist eine Verbindung von dem Schlauchanschlussstutzen 18 zum Inneren des Innenschaftes 12 hergestellt, so dass das freie Lumen im Inneren des Innenschaftes 12 als zweiter Spülkanal verwendet werden kann. Da der Innenschaft 12 als Spülkanal genutzt wird, ist die Öffnung 28 durch ein Dichtelement in Form einer auf das proximalseitige Ende des Innenschaftes 12 aufgesetzten Dichtkappe nach außen hin abgedichtet, wenn ein Instrument in den Innenschaft 12 eingesetzt ist. Befindet sich im Innenschaft 12 dagegen kein Instrument so erfolgt die Abdichtung über eine Membrandichtung 28a (siehe auch Figur 3).

Der Außenschaft 4 kann über den Schnellspannverschluss 8 leicht von dem Optikgehäuse 2 getrennt und durch einen anderen Außenschaft 4 ersetzt werden. Dazu kann der Außenschaft 4 von dem Optikschaft 26 und dem Innenschaft 12 abgezogen werden. Ferner kann der Innenschaft 12 über das Kupplungselement 30 ebenfalls leicht von dem Optikgehäuse 2 getrennt werden und nach hinten, d. h. in proximaler Richtung aus dem Optikgehäuse 2 entnommen werden. So ist es möglich, den Innenschaft 12 und den Außenschaft 4 gegen anders dimensionierte Außen- und Innenschäfte 4,12 auszutauschen. Da die Dichtung und Verbindung von Außenschaft 4 und Innenschaft 12 zu dem Optikgehäuse 2 über einen standardisierten Schnellspannverschluss 8 bzw. ein standardisiertes Kupplungselement 30 erfolgt, welche unabhängig vom Durchmesser der jeweiligen Außen- und Innenschäfte ausgebildet sind, ist es möglich, unterschiedlich dimensionierte Au-ßen- und Innenschäfte dichtend mit dem Optikgehäuse 2 zu verbinden. Bei allen Querschnittsgrößen von Außen- und Innenschaft sind die jeweiligen Anschlussteile, d. h. Kupplungselement 30 und Schnellspannverschluss 8 zur Verbindung mit dem Optikgehäuse 2 gleich ausgebildet.

Fig. 3 zeigt eine Einzelansicht des in Fig. 2 eingesetzten Innenschaftes 12. Zwischen den Umfangsdichtungen 32 sind sich radial erstreckende Öffnungen 36 angeordnet, welche einen Zugang zum Inneren des Innenschaftes 12 ermöglichen, um eine Spülflüssigkeitzu- und/oder abzuführen. Am proximalseitigen bzw. hinteren Ende des Innenschaftes 12 ist das Kupplungselement 30 zur Verbindung mit einem Optikgehäuse 2 ausgebildet. Das Kupplungselement 30 weist einen Kupplungskegel sowie einen Verschlussring zum verriegelnden und dichtenden Verbinden mit dem Optikgehäuse auf. Es kann somit in bekannter Weise eine dichte und leicht lösbare Verbindung hergestellt werden. Ferner ist am proximalen Ende des Innenschaftes 12 eine durch eine Dichtkappe abgedichtete Öffnung 28 vorgesehen, durch die Instrumente in den Instrumentenkanal, welcher durch den Innenschaft 12 definiert wird, eingeführt werden können. Ferner ist in dem Kupplungselement 30 eine Membrandichtung 28a vorgesehen, so dass keine Spülflüssigkeit am hinteren bzw. proximalen Ende aus dem Innenschaft 12 austreten kann. Bei der in dem Kupplungselement 30 zusätzlich angeordneten Membrandichtung 28a handelt es sich um einen geschlitzte Dichtscheibe, die im Bedarfsfall leicht ausgetauscht werden kann, indem der die Dichtkappe 44 tragende Instrumententeil 43 aus dem Kupplungselement 30 herausgeschraubt wird..

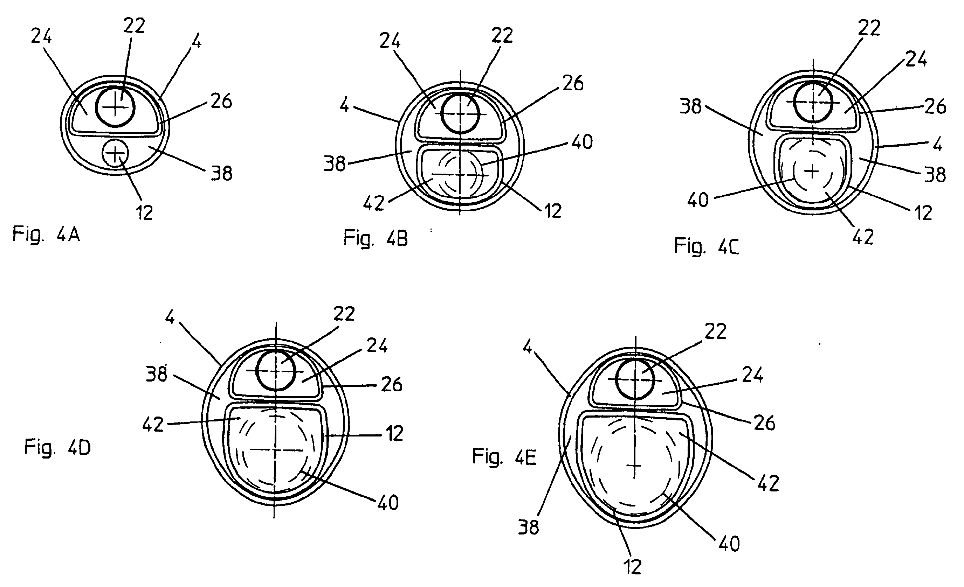

Die Figuren 4A bis 4E zeigen den Endoskopschaft, d. h. Außenschaft 4, Innenschaft 12 sowie Optikschaft 26 des anhand von Figuren 1 bis 3 erläuterten Endoskops im Querschnitt. In den Figuren 4A bis 4E sind jeweils unterschiedlich große Außenschäfte 4 und Innenschäfte 12 angeordnet. Der Optikschaft 26 ist immer derselbe, d. h. er weist einen konstanten Querschnitt auf.

Fig. 4A zeigt eine Anordnung, bei welcher ein Außenschaft 4 ohne einen Innenschaft verwendet wird. Der Außenschaft 4 bildet direkt einen Instrumentenkanal, in den ein Instrument 37 eingeführt ist. Der Querschnitt bzw. Innendurchmesser des Außenschaftes 4 wird hier im Wesentlichen durch die Außenabmessungen des Optikschafts 26 vorgegeben. Der Optikschaft 26 ist im Wesentlichen halbkreisförmig ausgebildet. Im zentralen Bereich des Optikschafts 26 ist das Bildbündel bzw. das Bildübertragungselement 22 zur Bildübertragung angeordnet. Seitlich davon ist das Lichtleitelement bzw. sind die Lichtleitfasern 24 zur Beleuchtung angeordnet. Der Optikschaft 26 füllt in etwa die Hälfte des Innenquerschnitts des Außenschaftes 4 aus. Der verbleibende Bereich zwischen dem Außenschaft 4 und dem Optikschaft 26 bildet einen Instrumentenkanal und gleichzeitig einen ersten Spülkanal 38. Der Innenquerschnitt bzw. Innendurchmesser des Außenschaftes 4 ist so gewählt, dass er gerade die Aufnahme des Optikschaftes 26 und des Instruments 37 ermöglicht. Auf diese Weise wird ein Endoskopschaft bzw. Außenschaft 4 mit minimalem Querschnitt bzw. Außendurchmesser geschaffen Bei dieser Anordnung steht nur ein Spülkanal zur Verfügung, so dass ein Dehnmedium intermittierend zu- und abgeführt wird.

Fig. 4B zeigt einen Querschnitt des Endoskopschaftes mit einem Außenschaft 4 und einem eingesetzten Innenschaft 12. Der Innenschaft 12 ist im Querschnitt im Wesentlichen halbkreisförmig ausgebildet. Gegenüber dem in Fig. 4A gezeigten Außenschaft 4 weist der Außenschaft 4 gemäß Fig. 4B einen größeren Querschnitt auf, so dass er gerade den Optikschaft 26 und den Innenschaft 12 in seinem Inneren aufnehmen kann. Dabei ist die Querschnittsform des Außenschaftes 4, weiche hier im Wesentlichen kreisförmig ist, derart gewählt, dass seitlich des Optikschaftes 26 und des Innenschaftes 12 ein Freiraum 38 verbleibt. Dieser Freiraum 38 kann, wie anhand von Fig. 4A erläutert, als Spülkanal 38 verwendet werden. Der Optikschaft 26 ist identisch zu dem Optikschaft 26 gemäß Fig. 4A , d. h. er weist denselben Querschnitt auf. In das Innere des Innenschaftes 12 kann ein Arbeitsgerät bzw. Instrument 40 eingeführt werden. Umfänglich des Arbeitsgerätes bzw. Instrumentes 40 verbleibt im Inneren des Innenschaftes 12 ein Freiraum 42, welcher als Spülkanal dient. Beispielsweise kann der Freiraum 42 zur Zufuhr einer Spülflüssigkeit genutzt werden, während der Freiraum 38 zur Abfuhr der Spülflüssigkeit dient.

Figuren 4C, 4D und 4E zeigen weitere Anordnungen entsprechend der Anordnung in Fig. 4B, wobei die Außenschäfte 4 und Innenschäfte 12 in jeder der Figuren andere Querschnittsgrößen aufweisen. Der Optikschaft 26 ist dagegen in allen Figuren identisch, d. h. er weist einen identischen Querschnitt auf. Die Figuren 4A bis 4E verdeutlichen, wie bei Verwendung eines stets gleichbleibenden Optikschaftes 26 unterschiedlich große Innenschäfte 12, d. h. Innenschäfte 12 mit unterschiedlichen Querschnitten, eingesetzt werden können. Die Verwendung unterschiedlich großer Innenschäfte 12 ermöglicht den Einsatz von Instrumenten 40 mit unterschiedlich großem Querschnitt. Der Querschnitt der Außenschöfte 4 wird entsprechend angepasst. Dabei wird die Querschnittsgröße bzw. Querschnittsform des Außenschaftes 4 so gewählt, dass seine Außenabmessungen vorzugsweise dem bei vorgegebenem Querschnitt des Innenschattes 12 und des Optikschaftes 26 minimalen Durchmesser entsprechen. Ferner können Außenschaft 4 und Innenschaft 12 so dimensioniert werden, dass der Freiraum 38 zwischen dem Innenschaft 12 und dem Optikschaft 26 sowie dem Außenschaft 4 eine gewünschte Querschnittsfläche aufweist. So kann auch die Querschnittsgröße des Spülkanals 38 genau eingestellt werden, um bestimmte Durchflussraten zu ermöglichen. Ferner kann durch Wahl der Querschnittsform des Innenschaftes 12 bei vorgegebenem Außendurchmesser eines Instruments 40 die Größe des Freiraums 42 im Inneren des Innenschaftes 12 gezielt beeinflusst werden, um einen zweiten Spülkanal mit definierter Querschnittsfläche zu erzielen. Wie anhand von Fig. 1 und Fig. 2 beschrieben, weisen alle Außenschäfte 4, welche unterschiedliche Querschnittsgrößen und/oder-formen aufweisen können, untereinander eine einheitliche bzw. gleich ausgebildete und dimensionierte Kupplungseinrichtung bzw. einen Schnellspannverschluss 8 auf, mit dem sie an einem Optikgehäuse 2 befestigt werden können. Auch die unterschiedlichen Innenschäfte 12 weisen untereinander unabhängig von Ihrer Querschnittsform und -größe einheitliche Kupplungs- bzw. Anschlusselemente 30 zur Verbindung mit dem Optikgehäuse 2 auf. Dies ermöglicht, eine Vielzahl unterschiedlicher Innenschäfte 12 und zugehöriger Außenschöfte 4 mit ein und demselben Optikgehäuse 2 zu verbinden, so dass sehr leicht unterschiedlich große Arbeits- und Spülkanäle zur Verfügung gestellt werden können, ohne eine Vielzahl unterschiedlicher Endoskope bereitzuhalten.

### Bezugszeichenliste

- 2: - Optikgehäuse
- 4: - Außenschaft
- 6: - distales Ende des Außenschaftes 4
- 8: - Schnellspannverschluss
- 10: - Okular
- 12: - Innenschaft
- 14: - Lichtleitkabelanschlussstutzen
- 16,18: - Schlauchanschlussstutzen
- 20: - Absperrhahn
- 22: Bildbündel bzw. Bildfaser
- 24: - Lichtleitfaser
- 26: - Optikschaft
- 28: - Öffnung
- 28a: Membrandichtung
- 30: - Kupplungselement
- 32: - Umfangsdichtungen
- 34: - Umfangsraum
- 36: - radiale Öffnungen
- 38: - Freiraum bzw. Spülkanal
- 40: - Instrument
- 42: - Freiraum
- 43: Schraubteil
- 44: Dichtkappe

## Patentansprüche

1. Endoskop mit einem an einem proximalen Ende vorgesehenen Optikgehäuse (2),
einem mit dem Optikgehäuse (2) lösbar verbundenen Außenschaft (4) und
einem Innenschaft (12), welcher im Inneren des Außenschaftes (4) parallel zu diesem verläuft und aus dem Außenschaft (4) entnehmbar ist **dadurch gekennzeichnet, dass** der Innenschaft (12) mit dem Optikgehäuse (2) lösbar verbunden ist.

2. Endoskop nach Anspruch 1, bei welchem im Inneren des Außenschaftes (4) ein Optikschaft (26) angeordnet ist, welcher sich in Längsrichtung des Außenschaftes (4) erstreckt und vorzugsweise mit dem Optikgehäuse (2) lösbar verbunden ist.

3. Endoskop nach Anspruch 2, bei welchem in dem Optikschaft (26) ein Lichtleiter (24) zur Beleuchtung und/oder eine Bildübertragungsoptik (22) angeordnet ist.

4. Endoskop nach einem der vorangehenden Ansprüche, bei welchem zwischen Innenschaft (12) und einer Innenwandung des Außenschaftes (4) und/oder zwischen dem Optikschaft (26) und einer Innenwandung des Außenschaftes (4) ein Freiraum (38) ausgebildet ist.

5. Endoskop nach einem der vorangehenden Ansprüche, bei welchem der Außenschaft (4) und der Innenschaft (12) über Kupplungskegelverbindungen (8,30) mit dem Optikgehäuse (2) lösbar verbunden sind.

6. Endoskopset, umfassend ein Endoskop nach einem der vorangehenden Ansprüche mit zumindest einem Außenschaft (4), welcher lösbar mit dem Optikgehäuse (2) verbindbar ist, und zumindest einem Innenschaft (12), welcher im Inneren des Außenschafts (4) anordbar und mit dem Optikgehäuse (2) lösbar verbindbar ist.

7. Endoskopset nach Anspruch 6, bei welchem der Innenschaft (12) im Inneren des Außenschaftes (4) derart anordbar ist, dass er exzentrisch zu dem Außenschaft (4) verläuft.

8. Endoskopset nach Anspruch 6 oder 7, bei welchem mehrere austauschbare Innenschäfte (11) vorgesehen sind, welche vorzugsweise unterschiedliche Querschnittsgrößen aufweisen.

9. Endoskopset nach einem der Ansprüche 6-8, bei welchem mehrere austauschbare Außenschäfte (4) vorgesehen sind, welche vorzugsweise unterschiedliche Querschnittsgrößen aufweisen.

10. Endoskopset nach einem der Ansprüche 6 bis 9, bei welchem ein Optikschaft (26) vorgesehen ist, welcher im Inneren des Außenschaftes (4) anordbar ist und vorzugsweise mit dem Optikgehöuse (2) verbunden ist oder mit dem Optikgehäuse (2) lösbar verbindbar ist.

11. Endoskopset nach Anspruch 10, bei welchem der Optikschaft (26) eine Querschnittsgröße aufweist, welche geringer ist als ein Innenquerschnitt jedes Außenschaftes (14).

## Claims

1. An endoscope with an optics housing (2) provided at a proximal end,
with an outer shank (4) releasably connected to the optics housing (2) and
with an inner shank (12) which in the inside of the outer shank (4) runs parallel to this and may be removed from the outer shank (4), **characterised in that** the inner shank (12) is releasably connected to the optics housing (2).

2. An endoscope according to claim 1, with which an optics shank (26) is arranged in the inside of the outer shank (4) and extends in the longitudinal direction of the outer shank (4) and is preferably releasably connected to the optics housing (2).

3. An endoscope according to claim 2, with which a fibre-optic (24) for illumination, and/or picture transmission optics (22) are arranged in the optics shank (26).

4. An endoscope according to one of the preceding claims, with which a free space (38) is formed between the inner shank (12) and an inner wall of the outer shank (4) and/or between the optics shank (26) and an inner wall of the outer shank (4).

5. An endoscope according to one of the preceding claims, with which the outer shank (4) and the inner shank (12) are releasably connected to the optics housing (2) via coupling-cone connections (8, 30).

6. An endoscope set comprising an endoscope according to one of the preceding claims with at least one outer shank (4) which is releasably connectable to the optics housing (2), and at least one inner shank (12) which may be arranged in the inside of the outer shank (4) and is releasably connectable to the optics housing (2).

7. An endoscope set according to claim 6, with which the inner shank (12) may be arranged in the inside of the outer shank (4) in a manner such that it runs eccentrically to the outer shank (4).

8. An endoscope set according to claim 6 or 7, with which several exchangeable inner shanks (11) are provided which preferably have different cross-sectional sizes.

9. An endoscope set according to one of the claims 6-8, with which several exchangeable outer shanks (4) are provided which preferably have different cross-sectional sizes.

10. An endoscope set according to one of the claims 6 to 9, with which an optics shank (26) is provided which may be arranged in the inside of the outer shank (4) and preferably is connected to the optics housing (2) or is releasably connectable to the optics housing (2).

11. An endoscope set according to claim 10, with which the optics shank (26) has a cross-sectional size which is smaller than an inner cross section of each outer shank (14).

## Revendications

1. Endoscope comprenant un boîtier d'optique (2) prévu à une extrémité proximale,
une tige extérieure (4) reliée de façon amovible au boîtier d'optique (2) et
une tige intérieure (12), qui est agencée à l'intérieur de la tige extérieure (4), parallèlement à celle-ci, et peut être enlevée de la tige extérieure (4), **caractérisé en ce que** la tige intérieure (12) est reliée de façon amovible au boîtier d'optique (2).

2. Endoscope selon la revendication 1, sur lequel est disposée à l'intérieur de la tige extérieure (4) une tige d'optique (26), laquelle s'étend dans la direction longitudinale de la tige extérieure (4) et, de préférence, est reliée de façon amovible au boîtier d'optique (2).

3. Endoscope selon la revendication 2, sur lequel un guide de lumière (24) pour l'éclairage et/ou une optique de transmission d'image (22) est disposés dans la tige d'optique (26).

4. Endoscope selon l'une des revendications précédentes, sur lequel un espace libre (38) est ménagé entre la tige intérieure (12) et une paroi intérieure de la tige extérieure (4) et/ou entre la tige d'optique (26) et une paroi intérieure de la tige extérieure (4).

5. Endoscope selon l'une des revendications précédentes, sur lequel la tige extérieure (4) et la tige intérieure (12) sont reliées de façon amovible au boîtier d'optique (2) au moyen d'assemblages coniques d'accouplement (8, 30).

6. Jeu d'éléments d'endoscopie comprenant un endoscope selon l'une des revendications précédentes avec au moins une tige extérieure (4), qui peut être reliée de façon amovible au boîtier d'optique (2), et au moins une tige intérieure (12), qui peut être disposée à l'intérieur de la tige extérieure (4) et peut être reliée de façon amovible au boîtier d'optique (2).

7. Jeu d'éléments d'endoscopie selon la revendication 6, dans lequel la tige intérieure (12) peut être disposée à l'intérieur de la tige extérieure (4) de telle sorte qu'elle soit agencée de façon excentrée par rapport à la tige extérieure (4).

8. Jeu d'éléments d'endoscopie selon la revendication 6 ou 7, dans lequel sont prévues plusieurs tiges intérieures (11) remplaçables qui présentent, de préférence, des tailles de section transversale différentes.

9. Jeu d'éléments d'endoscopie selon l'une des revendications 6 à 8, dans lequel sont prévues plusieurs tiges extérieures (4) remplaçables qui présentent, de préférence, des tailles de section transversale différentes.

10. Jeu d'éléments d'endoscopie selon l'une des revendications 6 à 9, dans lequel est prévue une tige d'optique (26), qui peut être disposée à l'intérieur de la tige extérieure (4) et, de préférence, est reliée au boîtier d'optique (2) ou peut être reliée de façon amovible au boîtier d'optique (2).

11. Jeu d'éléments d'endoscopie selon la revendication 10, dans lequel la tige d'optique (26) présente une taille de section transversale qui est inférieure à une section transversale intérieure de chaque tige extérieure (14).
